(19)

Europäisches
Patentamt
European
Patent Office
Office européen
des brevets

(11) **EP 3 797 305 B1**

(12) **EUROPEAN PATENT SPECIFICATION**

(45) Date of publication and mention
of the grant of the patent:
**19.02.2025 Bulletin 2025/08**

(21) Application number: **19734727.1**

(22) Date of filing: **21.06.2019**

(51) International Patent Classification (IPC):
**A61B 5/0538** (2021.01)    **A61B 5/00** (2006.01)
**G01R 27/02** (2006.01)

(52) Cooperative Patent Classification (CPC):
**G01R 27/02; A61B 5/0538; A61B 5/4094;**
A61B 5/24; A61B 5/291; A61B 5/4064

(86) International application number:
**PCT/EP2019/066518**

(87) International publication number:
**WO 2019/243596 (26.12.2019 Gazette 2019/52)**

(54) **SYSTEM AND METHOD FOR THE ESTIMATION OF PHYSICAL PARAMETERS OF A MEDIUM**

SYSTEM UND VERFAHREN ZUR SCHÄTZUNG PHYSIKALISCHER PARAMETER EINES
MEDIUMS

SYSTÈME ET PROCÉDÉ D'ESTIMATION DE PARAMÈTRES PHYSIQUES D'UN MILIEU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.06.2018 EP 18305801**

(43) Date of publication of application:
**31.03.2021 Bulletin 2021/13**

(73) Proprietors:
• **Université de Rennes**
**35042 Rennes (FR)**
• **Institut National de la Santé et de la
Recherche Médicale (INSERM)**
**75654 Paris Cedex 13 (FR)**

(72) Inventors:
• **MODOLO, Julien**
**35450 Dourdain (FR)**
• **CARVALLO, Andres**
**35000 Rennes (FR)**
• **WENDLING, Fabrice**
**35235 Thorigné-Fouillard (FR)**
• **BENQUET, Pascal**
**35160 Montfort-sur-Meu (FR)**

(74) Representative: **Icosa
83 avenue Denfert-Rochereau
75014 Paris (FR)**

(56) References cited:
CH-A2- 706 854        US-A- 5 489 849
US-A1- 2014 303 691    US-A1- 2016 089 051

• ITKIS M ET AL: "The square-wave approach to
impedance measurement of brain tissue water
dynamics", JOURNAL OF NEUROSCIENCE
METH, ELSEVIER SCIENCE PUBLISHER B.V.,
AMSTERDAM, NL, vol. 59, no. 2, 31 July 1995
(1995-07-31), pages 237 - 244, XP004586258,
ISSN: 0165-0270, DOI: 10.1016/0165-0270(94)
00209-Y
• BUTSON C R ET AL: "Sources and effects of
electrode impedance during deep brain
stimulation", CLINICAL NEUROPHYSIOLOGY,
ELSEVIER SCIENCE, IE, vol. 117, no. 2, 28
February 2006 (2006-02-28), pages 447 - 454,
XP028040551, ISSN: 1388-2457, [retrieved on
20060201], DOI: 10.1016/J.CLINPH.2005.10.007

**Description**

**FIELD OF INVENTION**

The present invention pertains to the field of measurement of physical parameters of a medium, notably a biological medium. In particular, the invention relates to a method for biophysical modelling of biological medium electrical conductivity using local pulsed electrical stimulation.

**BACKGROUND OF INVENTION**

The estimation of biological tissue electrical impedance has sparked interest in medical fields like oncology for diagnostic purposes. Healthy cells maintain a high concentration of potassium and a low concentration of sodium while one feature of cancerous cells is their lower cell membrane potential. Indeed, in injured or cancerous cell, sodium and water flows into the cell, decreasing potassium and other ions concentration in the intracellular medium, resulting in decreased impedance. Several studies have shown that healthy cells have higher electrical conductivity than tumor cells, given that cancer cells have different electrical and metabolic properties due to abnormalities in structures.

In addition to oncology, electrical conductivity measures have been performed in the field of muscular diseases to attempt differentiating healthy and diseased tissue based on conductivity.

Many studies reported potential clinical value of electrical conductivity in the field of oncology and muscular diseases. In contrast, there were only few attempts to evaluate the potential clinical/diagnostic value of electrical conductivity to characterize brain tissue in neurological disorders, such as epilepsy.

Epilepsy is a chronic neurological disorder affecting about 1% of the general population and is characterized by an altered balance between excitatory and inhibitory processes in brain circuits. It is defined by recurrent, chronic seizures interfering with functions such as language or motricity for example. Drug-refractory patients, accounting for approximately 30% of epileptic patients, can be considered for resective surgery of epileptogenic regions. In this context, pre-surgical planning frequently involves invasive recordings such as stereotactic electroencephalography (SEEG), consisting in the intracranial implantation of multiple electrodes recording electrophysiological signals in a large number of brain regions (typically 150-250 contacts), with the objective of identifying the epileptogenic zone. Recent studies measured the bioelectrical impedance and conductivity as a possible indicator of brain tissue epileptogenicity.

To date, the main approach to measure electrical conductivity in biological tissues consists in applying a current of predefined frequency by means of at least two electrodes in contact with the biological tissues and measuring the potential in said biological tissues. For example, US 2014/303691 A1 discloses a device for controlling delivery of stimulation signals to excitable tissue, such as nerves and/or muscles.

One major limitation of existing invasive measures of brain tissue conductivity is the lack of accounting for the biophysical processes occurring at the electrode-brain tissue interface and for interaction mechanisms between the electric field and the brain tissue itself.

This interface effect may be particularly important when electrodes are implanted intracranially into brain tissue, with the presence of cerebrospinal fluid or gliosis at the interface between the electrodes and brain tissue. Therefore, the physical processes occurring at the interface have to be considered to provide accurate estimates of electrical conductivity.

In other fields, such as horticultural products, food materials or water, there is also a need for fast and reliable estimation of physical parameters, for example to allow the detection of the processing conditions or the quality of food.

In this context, the present application describes a system and a method able to provide a fast and reliable estimation of physical parameters, in particular the electrical conductivity, of a region of a medium. Such a system and a method may be applied to evaluate the physical parameters of biological tissue, in particular to identify potential changes in conductivity due to pathophysiological process in biological tissues, notably brain tissues qualified as epileptic (pathological hyperexcitability). More generally, such a system and a method may be applied to evaluate physical parameters of a medium, which may be for example a biological tissue, a horticultural product, a food material or water.

**SUMMARY**

The present invention relates to a system for the estimation of at least one physical parameter of a region of a biological and/or physiological medium comprising at least one electrolyte, said system comprising:

- at least two electrodes configured to come into contact with the region of the medium;
- a current generator configured to deliver to the electrodes a train of electric pulses of current, each electric pulse having a pulse duration;
- a computer-readable memory comprising at least one predefined analytic model of an electric potential as a function of time, receiving as inputs at least the current and the pulse duration and comprising at least one physical parameter of the medium to be estimated, wherein the predefined analytic model is obtained from the coupling of an analytical model of the electric field generated by the electrodes with a double layer model generated at the electrode-medium interface, said coupling accounting for contributions from the electrode-electrolyte interface;

- an acquisition unit comprising a signal amplifier configured to acquire and amplify an electric potential recorded by the electrodes; and
- a processor comprising:

  • a stimulation module configured to control the current generator so as to deliver at least one electric pulse during a stimulation duration;
  • an acquisition module configured to trigger an acquisition of an electric potential variation as a function of time during a time window comprised in the stimulation duration; and
  • a calculation module configured to receive the acquired electric potential variation as a function of time, fit the acquired electric potential variation as a function of time using the predefined analytic model retrieved from the computer-readable memory, and output a value of the physical parameter obtained from the fitting of the predefined analytic model.

The predefined analytic model obtained from the coupling of an analytical model of the electric field generated by the electrodes with a double layer model generated at the electrode-medium interface, said coupling accounting for contributions from the electrode-electrolyte interface, has the advantage of providing an explicit, analytical expression of brain tissue conductivity based on the recorded brain tissue response to pulse stimulation. The model-based method of the present invention offers a fast and reliable estimation of brain tissue electrical conductivity by accounting for contributions from the electrode- electrolyte interface. This method outperforms standard bioimpedance measurements since it provides absolute (as opposed to relative) changes in brain tissue conductivity.

According to one embodiment, the physical parameter is associated to the electrical resistance of the region of the medium in which the electrodes are intended to be located.

According to one embodiment, the electrodes are bipolar cylindrical or plate electrodes.

According to one embodiment, the medium is a biological tissue, notably brain tissue.

According to one embodiment, the electrodes are configured for insertion in a region of said biological tissue.

According to one embodiment, the stimulation module controls the current generator so as to deliver electrical pulses having a current that does not saturate the signal amplifier.

According to one embodiment, each electrical pulse is a biphasic charge-balanced electrical pulse.

According to one embodiment, the stimulation module controls the current generator so as to deliver a biphasic charge-balanced current. The interest of using biphasic charge-balanced current for charge injection to the medium is to avoid charge accumulation that may modify or damage the medium. This feature is of particular interest for the use *in vivo* of the system for the analysis of biological tissues since, the use of monophasic stimulation cause very quickly, in time periods of the order of a few minutes, damage to the tissue (i.e. cell death).

According to one embodiment, the biphasic charge-balanced electrical pulses have a square waveform.

According to one embodiment, the acquired electric potential variation is measured with a sampling frequency superior to 8 kHz.

According to one embodiment, the calculation module is configured to receive geometry specifications of the electrodes and use said geometry specifications of the electrodes and the electrical resistance of the region of the medium to calculate the electrical conductivity of the region of the medium.

According to one embodiment, the system is configured to compare the value of the physical parameter to at least one predefined threshold.

The present invention further relates to a method for the local estimation of at least one physical parameter of a region of a medium, said method comprising steps of:

- receiving a measurement of an electric potential variation as a function of time in a time window, during which at least one electrical pulse is delivered to the region of the medium by at least two electrodes configured to come into contact with the medium, wherein the or each electric pulse has a pulse duration;
- fitting the measurement of the electric potential variation as a function of time using a predefined analytic model of the electric potential as a function of time, wherein the predefined analytic model receives as inputs at least the current and the pulse duration and comprises at least one physical parameter of the region of the medium; wherein the predefined analytic model is obtained from the coupling of an analytical model of the electric field generated by the electrodes with a double layer model generated at the electrode-medium interface, said coupling accounting for contributions from the electrode-electrolyte interface; and
- outputting a value of the physical parameter obtained from the fitting of the predefined analytic model.

According to one embodiment, the physical parameter is associated to the electrical resistance of the region of the medium in which the electrodes are intended to be located. According to one embodiment, the method further comprises a step of receiving geometry specifications of the electrodes and using said geometry specifications of the electrodes and the electrical resistance of the region of the medium to calculate the electrical conductivity of the region of the medium.

According to one embodiment, the medium is a biological medium, notably brain tissues. According to one embodiment, the value of the physical parameter is compared to at least one predefined threshold.

According to one embodiment, the or each electrical pulse is a biphasic charge-balanced electrical pulse.

According to one embodiment, the electric potential variation received is measured with a sampling frequency superior to 8 kHz.

Yet another aspect of the present invention relates to a method for generating a mapping of a physical parameter of an area of a medium using at least two electrodes configured to come into contact with the medium, said method comprising steps of:

- receiving information concerning a first position of the electrodes in a first region of the medium comprised in the area of the medium being mapped;
- obtaining a first value of the physical parameter of the medium in the first region of the medium according to the method of any one of the embodiments described hereabove; and
- associating and registering the first position of the electrodes with the first value of the physical parameter;

wherein the steps of the method are repeated for at least one second position of the electrodes in a second region of the medium comprised in the area of the medium being mapped.

The present invention further relates to a computer program comprising instructions which, when the program is executed by a computer, cause the computer to carry out the steps of the method according to any one of the embodiments described hereabove.

The present invention further relates to a computer-readable medium comprising instructions which, when executed by a computer, cause the computer to carry out the steps of the method according to any one of the embodiments described hereabove.

**DEFINITIONS**

In the present invention, the following terms have the following meanings:

- As used herein the singular forms "a", "an", and "the" include plural reference unless the context clearly dictates otherwise.

- **"Electroencephalogram"** or **"EEG"** refers to the tracing of brain waves, by recording the electrical activity of the brain from the scalp, made by an electroencephalograph.

- **"Double layer"** also called an **"electrical double layer",** refers to two parallel layers of charge surrounding an object at the surface. A first layer, the surface charge (either positive or negative), consists of ions adsorbed onto the object due to chemical interactions. A second layer is composed of ions attracted to the surface charge via the Coulomb force, electrically screening the first layer. This second layer is loosely associated with the object.

- **"Stereotactic Electroencephalography",** refers to an electroencephalography technic wherein the electrodes are implanted in the patient brain tissue by mean of stereotactic surgery, a minimally invasive form of surgical intervention which makes use of a three-dimensional coordinate system.

- **"Subject"** refers to a mammal, preferably a human. In the sense of the present invention, a subject may be an individual having any mental or physical disorder requiring regular or frequent medication or may be a patient, i.e. a person receiving medical attention, undergoing or having underwent a medical treatment, or monitored for the development of a disease.

**BRIEF DESCRIPTION OF THE DRAWINGS**

**Figure 1** is a schematic representation of a system according to one embodiment of the invention for the estimation of at least one physical parameter (P) of a region of a medium (M).

**Figure 2** is a schematic representation of a system according to one particular embodiment of the invention where the electrodes are cylindrical electrodes for brain tissue.

**Figure 3 (a)** is a schematic representation of the ionic distribution in the double layer model.

**Figure 3 (b)** is the equivalent circuit for a single electrode-electrolyte interface, according to one embodiment.

**Figure 4** is the two-electrode double layer circuit model, where Rm models the medium resistance, according to one embodiment.

**Figure 5** is a representation of the electric potential induced in a medium as a function of time in response to a single stimulation pulse of intensity, according to one embodiment.

**Figure 6** is a block diagram of the method of the present invention, according to one embodiment.

**Figure 7** is a block diagram of the method of the present invention, according to one embodiment comprising a step of calculating the electrical conductivity of the medium.

**Figure 8** is a schematic representation of a depth electrode.

**Figure 9** is a schematic representation of intracranial electrode geometry. Subfigure 9 (a) is an illustration of a pair of contacts for a bipolar cylindrical electrode used clinically, considering that the electrode is oriented along the z-axis, and that each contact at the outer surface of the electrode of radius R has a height h. The considered pair of contacts is separated by a distance 1. Subfigure 9 (b) shows the differential ring contribution in a cylindrical electrode, enabling accounting for the geometry of the electrodes.

**Figure 10** concerns predefined analytic model parameters estimation for Patient 1. Subfigure 10 (a) shows the signals waveform for two different electrodes, illustrating the amplitude difference between regions identified as epileptic and healthy, respectively. Subfigure 10 (b) is a box plot showing the estimated conductivity for each stimulated brain region. Subfigure 10 (c) is a box plot showing the estimated double layer capacitance. Subfigure 10 (d) is a box plot showing the estimated faradaic impedance.

**Figure 11** concerns predefined analytic model parameters estimation for Patient 2. Subfigure 11 (a) shows the signals waveform for two different electrodes, illustrating the amplitude difference between regions identified as epileptic and healthy, respectively. Subfigure 11 (b) is a box plot showing the estimated conductivity for each stimulated brain region. Subfigure 11 (c) is a box plot showing the estimated double layer capacitance. Subfigure 11 (d) is a box plot showing the estimated faradaic impedance.

**Figure 12** shows the agreement of the predefined analytical model with recorded signals in saline solutions. The graphs represent superimposed time course of simulated (dotted line) and experimentally recorded (full line) electric potentials for four different conductivity values: (a) 0.1 S/m, (b) 0.2 S/m, (c) 0.4 S/m, and (d) 0.6/m.

## DETAILED DESCRIPTION

The following detailed description will be better understood when read in conjunction with the drawings. For the purpose of illustrating, the system and the method are shown in the preferred embodiments. It should be understood, however, that the application is not limited to the precise arrangements, structures, features, embodiments, and aspect shown. The drawings are not drawn to scale and are not intended to limit the scope of the claims to the embodiments depicted. Accordingly, it should be understood that where features mentioned in the appended claims are followed by reference signs, such signs are included solely for the purpose of enhancing the intelligibility of the claims and are in no way limiting on the scope of the claims.

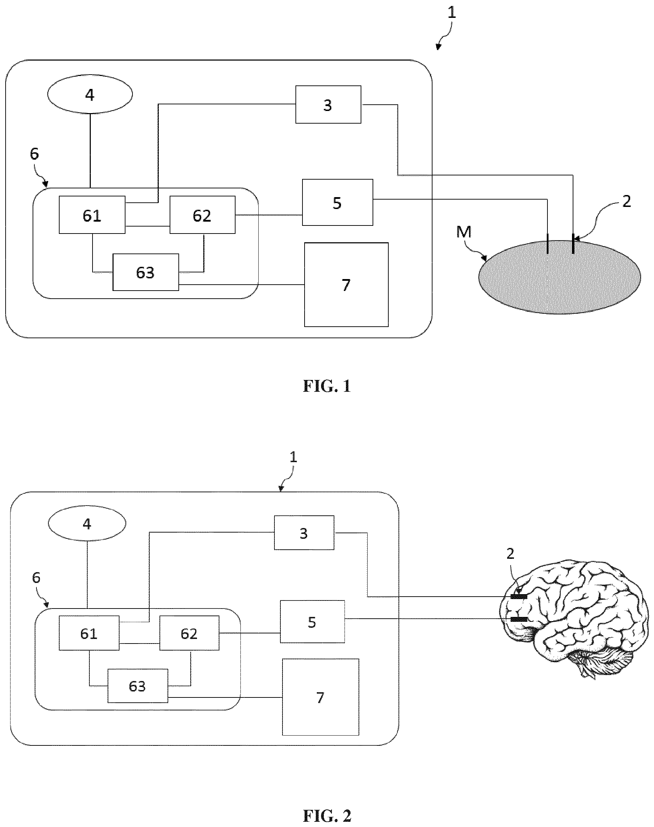

As shown in Figure 1, the system **1** according to one embodiment comprises at least two electrodes **2** configured to come into contact with a region of the medium under examination.

According to one embodiment, each electrode **2** comprises a main structure and at least an active area, also called in the present description "electrode contact".

According to one embodiment, at least a portion of the electrode active area is configured to come into contact with the medium.

According to a preferred embodiment, the electrode **2** is configured to be inserted in the medium so as to be totally surrounded by the medium in at least a first portion of the electrode, said first portion of the electrode comprising at least two electrode contacts.

In an exemplary situation with two electrodes inserted in the medium, one of the electrodes is typically termed "working electrode" and the other "counter electrode".

According to one embodiment, the number of electrode contacts configured to come into contact with the medium is greater than 2. In one example, the number of electrode contacts in the medium may range from 2 to 200. The electrodes may be independently displaceable in the medium or arranged in an array presenting a predefined spacing between the electrodes.

The electrodes **2** may have different geometries and dimensions depending on the medium under investigation. For example, when the analyzed medium is a liquid solution, electrodes may have a flat panel shape or a toroidal shape may be used, or any other suitable shape.

In the alternative example represented in Figure 2, where the medium to analyze is a region of the brain, the electrodes have a cylindrical shape with a small cross section in order to be easily and safely inserted into the cerebral tissues. According to this example, the electrodes are bipolar cylindrical electrodes.

According to one embodiment, the system **1** further comprises a current generator **3** configured to deliver throughout the electrodes **2** a train of electric pulses of current **I**, wherein each electric pulse has a pulse duration **T.** The current generator **3** may be attached between the working electrode(s) and the counter electrode(s).

According to one embodiment, the current generator 3 is configured to deliver a charge-balanced biphasic pulsing, wherein a constant current is passed in one direction during a pulse duration **T,** and then is reversed during the same time **T,** such that the delivered charge during each pulse phase is strictly the same (same intensity by duration product, i.e. charge).

The interest of using current-controlled pulses for charge injection to the medium is to avoid charge accumulation that can damage the medium. This feature is of particular interest for the use of the system is the *in vivo* analysis of biological tissues such as brain tissue.

According to one embodiment, the system **1** further comprises a computer-readable memory **4** comprising at least one predefined analytic model **M(t)** of an electric potential as a function of time between the working electrode and the counter electrode. In this embodiment, said predefined analytic model **M(t)** is defined so as to receive as inputs at least the current **I** and the pulse duration **T** and comprises at least one physical parameter **P** of the medium **M** that has to be measured.

According to one embodiment, the system **1** further comprises an acquisition unit **5** comprising a signal amplifier configured to acquire and amplify the value of the electric potential recorded by the electrodes **2,** notably the electric potential recorded between the working electrode and the counter electrode. According to one embodiment, the sampling frequency of the acquisition unit **5** is superior to 8 kHz, preferably the sampling frequency is comprised in the [25 - 100 kHz] range. According to one embodiment, the current **I** delivered by the electrical pulses is chosen in order to not saturate the amplifier.

According to one embodiment, the system **1** comprises a processor **6** comprising multiple modules configured to communicate with and control the current generator **3,** computer-readable memory **4** and the acquisition unit **5.**

According to one embodiment, the processor **6** comprises a stimulation module **61,** an acquisition module **62** and a calculation module **63.**

According to one embodiment, the stimulation module **61** is configured to control the current generator **3** so as to deliver at least one electric pulse during a stimulation duration **Ts.**

According to one embodiment, the acquisition module **62** is configured to trigger an acquisition of an electric potential variation as a function of time **ΔV(t)** during a time window **Tm** comprised in the stimulation duration **Ts.**

According to one embodiment, the calculation module **63** is configured to receive the acquired electric potential variation as a function of time **ΔV(t)**, fit the acquired electric potential variation as a function of time **ΔV(t)** using the predefined analytic model **M(t)** retrieved from the computer-readable memory, and output a value of the physical parameter **P** obtained from the fitting of the predefined analytic model **M(t)**. The calculation module **63** may further register the value of the physical parameter **P** in the computer-readable memory **4.**

According to one embodiment, multiple predefined analytic model **M(t),** each describing different geometries of electrodes and/or different characteristics and geometries of the medium to analyze, are stored in the computer-readable memory **4.** According to this embodiment, the system **1** further receives as input at least a user choice concerning the predefined analytic model **M(t)** that has to be retrieved by the calculating module **63.**

According to one embodiment, the system **1** further comprises a user interface **7** configured to display the acquired electric potential variation as a function of time **ΔV(t)** and/or the output of the calculation module **63.**

Yet another aspect of the present invention relates to a method for the local estimation of at least one physical parameter **P** of a region of a medium **M.**

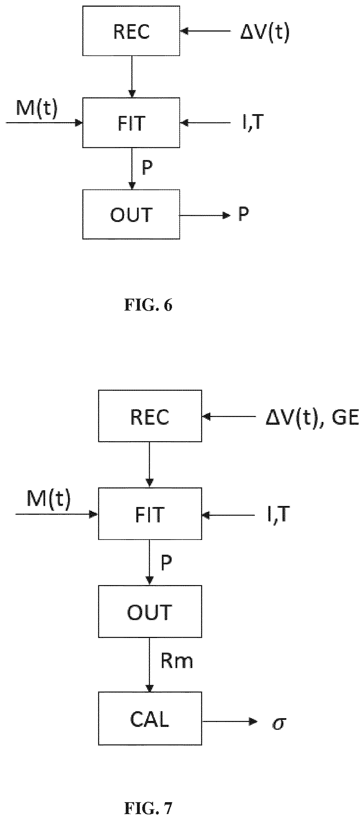

The steps of the method are represented in the block diagrams in Figures 6 and 7. According to one embodiment, the method comprises a preliminary step of receiving **REC** a measurement of an electric potential variation as a function of time Δ**V(t)** during a time window **Tm**. During said time window **Tm** at least one electrical pulse is delivered to the region of the medium **M** by at least two electrodes configured to come into contact with the medium **M.**

According to one embodiment, the or each electrical pulse is a biphasic charge-balanced electrical pulse having pulse duration **T** superior or equal to 0.06 ms.

According to one embodiment, this preliminary step REC is preceded by a measurement step consisting in the measurement during a time window **Tm** of the electric potential variation as a function of time Δ**V(t)** induced by the electrical pulse delivered to the region of the medium **M** by the electrodes. According to one embodiment, the measurement step is performed with a sampling frequency superior to 8 kHz, preferably the sampling frequency is comprised in the [25 - 100 kHz] range. This high sampling frequency is optimized in order to be able to record a good quality signal during a lapse of time of a few tens or hundreds of microseconds following the electric pulse, in order to obtain enough samples during the stimulation pulse.

The method of the present invention may further comprise the step of receiving, for example from a computer-readable medium, a predefined analytic model **M(t).**

Said predefined analytic model **M(t)** depends mainly on the geometry of the region of the medium and on the geometry and disposition of the electrodes. According to one embodiment, the electric field model, describing the electric field generated from the electrodes inside the medium, is derived from Maxwell's equations for a predefined geometry of the electrodes and geometry of the medium **M.**

From Maxwell's equations, the differential version of Ampere's law is:

$$\nabla \times \boldsymbol{B} = \mu_0 \boldsymbol{J} + \mu_0 \varepsilon_0 \frac{\partial \boldsymbol{E}}{\partial t} \qquad (1)$$

where **E** and **B** are the electric and magnetic field, respectively; **J** the current density; $\mu_0$ and $\varepsilon_0$ the electric permeability and permittivity of vacuum, respectively. One or several approximation(s) may be done, on the basis of the geometry of the electrodes or characteristics of the medium, in other to derive a simplified electric field model.

According to the embodiment where the pulse frequencies are lower than 10 kHz, a quasi-static approximation is used, i.e. $\frac{\partial E}{\partial t} = 0$ . Since the divergence of the curl for any vector field is null, i.e. $\nabla \cdot (\nabla \times \boldsymbol{B}) = 0$, we find $\nabla \cdot \boldsymbol{J} = 0$. Furthermore, as a first approximation, the medium may be considered as purely resistive, i.e. following the general form of Ohm's law $\boldsymbol{J} = \sigma \cdot \boldsymbol{E}$, where $\sigma$ is the electrical conductivity (expressed in Siemens per meter, [S/m]). Another assumption that may be done to develop an analytical expression of the medium response to pulsed stimulation is that the medium conductivity is locally isotropic, which simplifies the conductivity tensor into a scalar. Such an electric field model depends on the electrical conductivity of the medium and on the geometry of the electrodes.

Reduction-oxidation reactions takes place when a metal is placed into a physiological medium **M** (also called electrolyte). According to one embodiment, a model of the complex processes taking place during electrical stimulation of the medium is introduced in the predefined analytic model **M(t)** in order to describe and understand how the waveform of the delivered electric stimulus is altered by the physical properties of the medium **M.** This model enables the estimation of electrical conductivity from the recorded medium **M** response, while accounting for the contribution of the electrode-electrolyte interface to this response. The advantage of introducing a model of the processes taking place during electrical stimulation of the medium in the predefined analytic model **M(t)** is that of obtaining a biophysical model able to remove the contribution of the electrode-electrolyte interface to the response recorded from the medium **M,** and thus faithfully estimate the real medium (ex. brain tissue) response to the applied electric field.

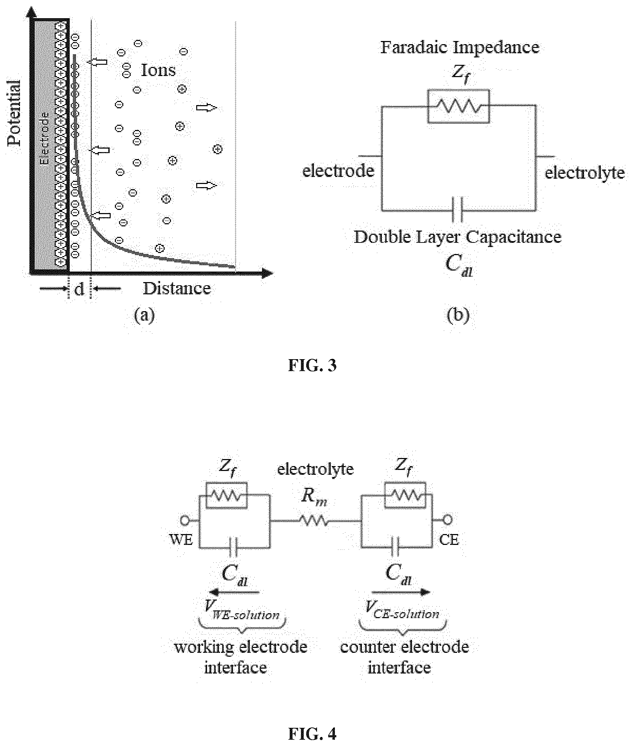

An assumption may be done that charge is also injected from the electrode to the electrolyte through Faradaic processes of reduction/oxidation, where electrons are transferred between the two phases. According to one embodiment shown in Fig. 3 (b), the electrical model for the electrode-electrolyte interface is modelled by a circuit comprising an impedance $\boldsymbol{Z_f}$ and a capacitance $\boldsymbol{C_{dl}}$ using a double layer model. In this model, $\boldsymbol{Z_f}$ is the Faradaic impedance representing Faradaic processes, whereas $\boldsymbol{C_{dl}}$ models the double layer capacitance, i.e. the ability of the electrode to cause charge flow in the electrolyte without electron transfer. The ionic distribution in the double layer model is shown in Figure 3 (a).

According to the embodiment where at least one working and at least one counter electrode are inserted into the medium **M,** a double layer model is used leading to a two-electrode double layer circuit model presented in Figure 4. This two-electrode double layer circuit model comprises two circuits, each circuit comprising a capacitance and a faradaic impedance connected in parallel, connected in series by a resistance **Rm,** modelling the medium resistance. A current source is attached between the working electrode and the counter electrode represented by the two impedance-capacitance circuits.

According to one embodiment, the electric potential expression between the working and counter electrode $V_{WE-}$

$_{CE}(s)$ is derived by solving the equivalent two-electrode double-layer circuit model using a Laplace transformation, leading to:

$$V_{WE-CE}(s) = \frac{2Z_f + R_m(1 + C_{dl}Z_f s)}{(1 + C_{dl}Z_f s)} I(s) \qquad (2)$$

where $I(s) = L\{I(t)\}$. According to the embedment wherein a charge-balanced biphasic electrical pulsing is delivered to the medium, $i(t)$ represent the stimulating biphasic pulse such that $i(t) = I[u(t) - 2u(t - T) + u(t - 2T)]$, $u(t)$ being the Heaviside function.

The Laplace transform of the current is $I(s) = \frac{I}{s}(1 - 2e^{-sT} + e^{-2sT})$, leading to:

$$V_{WE-CE}(s) = \frac{2Z_f + R_m(1 + C_{dl}Z_f s)}{(1 + C_{dl}Z_f s)} \frac{I}{s}(1 - 2e^{-sT} + e^{-s2T}) \qquad (3)$$

According to one embodiment, the inverse Laplace transform is used to obtain the predefined analytic model **M(t)** expression of the resulting electric potential in the time domain:

$$M(t) = I\left\{u(t)\left[R_m + 2Z_f\left(1 - e^{-\frac{t}{C_{dl}Z_f}}\right)\right]\ldots \qquad (4)$$
$$- 2u(t - T)\left[R_m + 2Z_f\left(1 - e^{-\frac{(t-T)}{C_{dl}Z_f}}\right)\right]\right\}$$

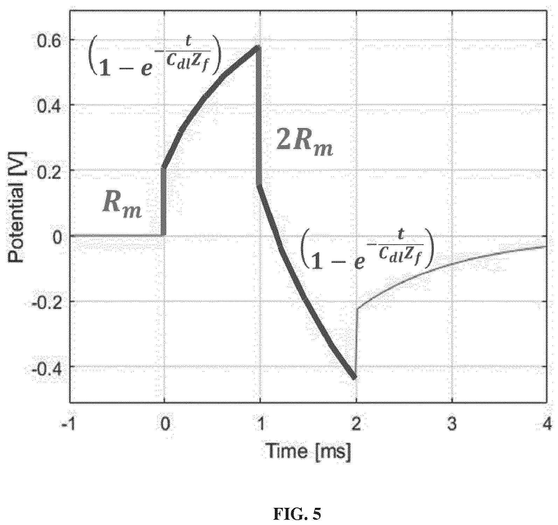

This provides an analytic model for the electric potential measured between the two electrode contacts during bipolar, charge-balanced current controlled stimulation. According to this embodiment, the inputs of the predefined analytic model **M(t)** are the current **I** and the pulse duration **T,** assumed equal for each phase (positive/negative) and the unknown physical parameters **P** are $C_{dl}$, $Z_f$ and $R_m$. Figure 5 presents a graph illustrating the resulting electric potential time course in response to a biphasic stimulation pulse, and how the model parameters impact the modeled response.

For an arbitrary geometry of electrodes, the medium resistance $R_m$ may be expressed as a function of the electric field **E** as:

$$R_m = \frac{V}{I} = \frac{\int E \cdot dl}{\oint J \cdot dS} = \frac{\int E \cdot dl}{\oint \sigma E \cdot dS} \qquad (5)$$

From this expression, the solution's medium resistance **Rm** only depends on medium geometry and conductivity $\sigma$. Using an electric field model depending on the geometry of the electrodes, an expression for the electrical resistance of the medium **Rm** may be derived. By combining an estimation of **Rm** and knowing electrode geometry specifications, and as consequence an appropriate electric field model generated by the electrodes in the medium can be developed, and the electrical conductivity may be estimated.

In the present model, the medium resistance **Rm** is expressed explicitly. Indeed, the medium resistance **Rm** expression is obtained from a system of three mathematical equations taking into account the electric field, the electrode-tissue interface and the biphasic pulses.

According to one embodiment, the method further comprises a step **FIT** consisting in the extrapolation of a physical parameter **P** by fitting the measurement to a predefined analytic model **M(t)** of the electric potential as a function of time. According to the embodiment, the unknown physical parameters **P** $C_{dl}$, $Z_f$ and $R_m$ are fitted to the measured values of electric potential variation Δ**V(t)**.

According to one embodiment, a minimum mean squared error estimator (MMSE) is used as an estimator. The MMSE is based on the estimation of the error between the estimated parameter and the actual parameter value as the basis for optimality. MMSE estimators as have the advantage of an easier implementation over optimal Bayesian estimators. The observation, i.e. the measured values of potential variation over time Δ**V(t)**, may be modeled as a nonlinear combination $f(t, P)$ of the model unknown physical parameters **P** $= [R_m ; C_{dl} ; Z_f]$ as follows:

$$\widehat{P} = \min_{C} E([\Delta V(t) - f(t,P)]^2) \qquad (6)$$

In general, there is no closed-form solution for (6). A nonlinear regression was used, starting with an initial value for the model parameter **P**.

According to one embodiment, once the model parameters $\widehat{P} = [\widehat{R_m}\,;\widehat{C_{dl}}\,;\widehat{Z_f}]$ are estimated, the electrical conductivity of the medium is computed **CAL** using the relationship between medium resistance **Rm** and electrical conductivity $\sigma$ that can be derived from equation (5) when the electric field model is known, i.e. when the geometry of the electrodes is defined.

According to one embodiment, the method comprises a step **OUT** of outputting the value of the physical parameter **P** obtained from the fitting of the predefined analytic model **M(t).**

According to one embodiment, the value of the physical parameter **P** is compared to a predefined threshold, said predefined threshold depending on the medium **M** under examination.

The main advantages of this model-based method are its accuracy and low computational cost.

According to one example, the system is configured for the estimation of at least one biophysical parameter **P** of a brain tissue region *in vivo.* The measurement of biophysical parameter **P** of a brain tissue region *in vivo,* such as electrical conductivity $\sigma$, can be used as an application for pre-surgical evaluation and identification of epileptogenic regions in patients with drug-refractory epilepsies.

The advantage of the approach herein proposed for the application to biological tissue is that of taking into account the biophysics of the tissue, indeed in the present approach the resistance of the medium **Rm** (related to the conductivity) is calculated semi-analytically from a biophysical model of the electric field induced by the electrodes and electrode-electrolyte interface.

The present invention goes beyond the prior art by proposing simple measures of a global bio-impedance of the tissue, where the electro-electrolyte interface effect is present and prevents any absolute measurement of the conductivity of the tissue (only relative).

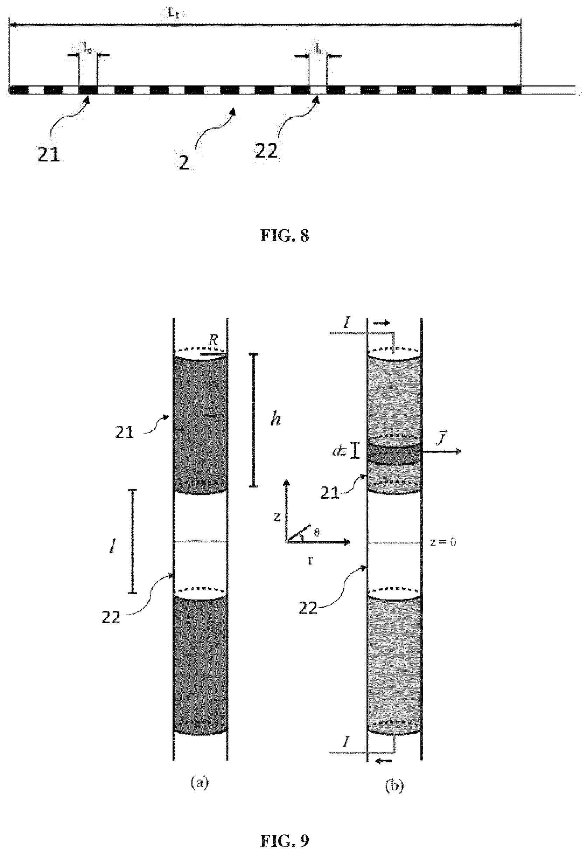

In this example, bipolar electrodes are depth electrodes configured for frame-based stereotactic implantation. Depth electrodes are gaining popularity due to the low complication rates reported for stereo-electroencephalography as compared to invasive monitoring using large craniotomies for grid and strip electrode implantation. One depth electrode **2** consists in an array of 10 to 15 cylindrical contacts **21** positioned apart with a pitch ranging from 0.5 mm to 5 mm and separated by insulating material **22** along one longitudinal direction, as shown in Figure 8. In this description it is considered that the depth electrode is oriented along the z-axis, and that each electrode contact **21** at the outer surface of the electrode of radius R has a height h. The electrode contacts of the considered pair of electrode contacts are separated by a distance l. These cylindrical contacts **21** may have a length ranging from 1 mm to 1 cm, diameter ranging from 0.5 mm to 2 mm and they may be made of platinium-iridium. Implanted under stereotactic conditions, they offer the advantage to probe multiple brain regions either cortical or sub-cortical. An example of such bipolar cylindrical electrode is reported in Figure 9 (a).

A cylindrical electric field model is used in order to model the electric field generated in the medium by these electrodes **2** having a cylindrical shape.

As shown in Figure 9 (b), a differential ring on a cylindrical electrode is approximated by a point source. A differential current *dI* induces an electric potential $dV(\rho) = dI/4\pi\sigma\rho$, where $\rho$ is the distance from the differential ring. By integrating the differential current *dI* along the height of the electrode contact **21,** and expressing the differential current as $dI = J\,2\pi Rd\zeta,$ the electric potential from a single electrode contact **21** is:

$$V = \frac{I}{4\pi\sigma h}\left[\sinh^{-1}\left(\frac{z-\frac{l}{2}}{r}\right) - \sinh^{-1}\left(\frac{z-\frac{l}{2}-h}{r}\right)\right] \qquad (7)$$

Considering the same expression for a second contact and using the superposition principle, the total electric potential induced by both electrode contacts **21** is:

$$V(r,z) = \frac{I}{4\pi\sigma h}\left[sinh^{-1}\left(\frac{z-\frac{l}{2}}{r}\right)\right.$$

$$\left. - sinh^{-1}\left(\frac{z-\frac{l}{2}-h}{r}\right) + sinh^{-1}\left(\frac{z+\frac{l}{2}}{r}\right) - sinh^{-1}\left(\frac{z+\frac{l}{2}+h}{r}\right)\right] \qquad (8)$$

Applying the gradient operator on the electric potential leads to the electric field model components in cylindrical

coordinates:

$$E_r(r,z) = \frac{I}{4\pi\sigma hr}\left[\frac{z-\frac{l}{2}}{\sqrt{r^2+(z-\frac{l}{2})^2}} - \frac{z-\frac{l}{2}-h}{\sqrt{r^2+(z-\frac{l}{2}-h)^2}} + \frac{z+\frac{l}{2}}{\sqrt{r^2+(z+\frac{l}{2})^2}} - \frac{z+\frac{l}{2}+h}{\sqrt{r^2+(z+\frac{l}{2}+h)^2}}\right] \qquad (9)$$

$$E_z(r,z) = \frac{I}{4\pi\sigma h}\left[\frac{1}{\sqrt{r^2+\left(z-\frac{l}{2}-h\right)^2}} - \frac{1}{\sqrt{r^2+\left(z-\frac{l}{2}\right)^2}} + \frac{1}{\sqrt{r^2+\left(z+\frac{l}{2}+h\right)^2}} - \frac{1}{\sqrt{r^2+(z+\frac{l}{2})^2}}\right] \qquad (10)$$

As described above, a model of the reduction-oxidation reaction is used to the estimation of electrical conductivity $\sigma$ from the recorded brain tissue response, while accounting for the contribution of the electrode-electrolyte interface to this response. In a clinical situation, at least two electrode contacts **21** are located in brain tissue (the electrolyte). The current-controlled pulse is often used for charge injection to the tissue to avoid charge accumulation that can damage brain tissue. A current source is attached between the working electrode contact and the counter electrode contact. Charge-balanced biphasic pulses are delivered to brain tissues throughout the electrode contact. The first phase is used, for example, to elicit the desired physiological effect such as initiation of an action potential, and the second phase is used to reverse electrochemical processes occurring during stimulation. In this example, the analytic model **M(t)** for the electric potential measured between the two electrode contacts **21** during bipolar, charge-balanced current controlled stimulation is expressed as in equation 4. A basic assumption of the model is that the amplitude of the stimulation artifact at the level of the tissue is considerably higher than the level of background neural activity. This assumption is justified by the difference in the amplitude of both signals (typically 70 $\mu$V for background activity versus approximately 1 V for the stimulation artifact). Using this reasonable assumption, the contribution of sources from neuronal activity in the Laplace equation is neglected.

An expression for the electrical resistance of the medium Rm is derived from the cylindrical electric field model of equations (9,10). Assuming that the electric potential difference measured is approximately equal to the difference between the potential at the border of the first electrode (at r =R) and the potential in the border of the second electrode, the cylindrical model leads to:

$$V(z) = \frac{I}{4\pi\sigma h}\left[sinh^{-1}\left(\frac{z-\frac{l}{2}}{R}\right) - sinh^{-1}\left(\frac{z-\frac{l}{2}-h}{R}\right) + sinh^{-1}\left(\frac{z+\frac{l}{2}}{R}\right) - sinh^{-1}\left(\frac{z+\frac{l}{2}+h}{R}\right)\right] \qquad (11)$$

$$\Delta V = V\left(\frac{1}{2}(l+h)\right) - V\left(-\frac{1}{2}(l+h)\right) \qquad (12)$$

$$\Delta V = \frac{I}{2\pi\sigma h}\left[2sinh^{-1}\left(\frac{h}{2R}\right) + sinh^{-1}\left(\frac{l+\frac{h}{2}}{R}\right) - sinh^{-1}\left(\frac{l+\frac{3h}{2}}{R}\right)\right] \qquad (13)$$

where the electrical resistance Rm can be expressed as:

$$R_m = \frac{1}{2\pi\sigma h}\left[2sinh^{-1}\left(\frac{h}{2R}\right) + sinh^{-1}\left(\frac{l+\frac{h}{2}}{R}\right) - sinh^{-1}\left(\frac{l+\frac{3h}{2}}{R}\right)\right] \qquad (14)$$

As an approximation, is used the mid-point ( $z = \frac{1}{2}(l+h)$ ) of the electrode contact in order to estimate the tissue resistance. In order to obtain an exact expression, it would be required to perform an integral along the z-axis to derive a mean electrical potential value. Therefore, this simplification was made since identifying such an analytical expression is highly complex (if possible at all). Considering another point of the electrode such as $z = \frac{1}{2}$ will induce a small bias in the estimation. By combining an estimation of **Rm** obtained from the fitting of the analytic model **M(t)** and knowing the electrode geometry specifications, the electrical conductivity $\sigma$ can be estimated.

The main advantages of this model-based method are its accuracy, low computational cost, and compatibility with stimulation hardware and parameters routinely used in clinics, making it immediately applicable.

In a typical clinical setting, electrical conductivity cannot be measured during stereotactic electroencephalography (SEEG) recordings as functional stimulation sessions aim at identifying the epileptogenic zone based on the analysis of after-discharges elicited by stimulation, like cortico-cortical evoked potentials. An advantage of the method in the present invention is the use of stimulation parameters **(I, T)** compatible with standard clinical stimulations performed prior in presurgical evaluation, even using lower stimulation intensity. Therefore, not only electrical conductivity can be estimated from electrophysiological recordings, but it is even safer than standard functional stimulation protocols (intensity between 5 and 25 times lower). In addition, while the traditional bioimpedance technique provides some contrast between healthy and epileptogenic regions, the method of the present invention has the advantage of providing absolute instead of relative estimates of electrical conductivity.

A further advantage of the method of the present invention is that the characteristics of the stimulation artifact, typically completely discarded since deemed as inexploitable, can be used to gain further knowledge of the biophysical properties of brain tissue, possibly providing information of diagnostic interest.

Electrical conductivity estimation could lead to the development of novel markers of "abnormal brain tissue" which can complement the analysis of SEEG intracerebral recordings classically performed prior to surgery.

In this example, the comparison of the value of the biophysical parameter **P** to a predefined threshold allows to discriminate the pathological from the healthy region of a subject's brain. In this case, the predefined threshold is established on the base of successive measurements of pathological and healthy regions in the brain of multiple subjects.

According to one embodiment, the method of the present invention is used to estimate the physical parameters of a biological tissue *in vivo* or *ex vivo.*

According to one embodiment, the method is used to detect variation in the physical parameters of an organ in order to evaluate the presence of metastatic and/or cancerous regions.

According to one embodiment, the method is used to detect variation in the physical parameters of brain tissues in order to identify the presence of pathological brain regions. According one embodiment of the present invention, the pathological brain regions arise from an epileptic condition.

The ILAE (International League Against Epilepsy) has published in 2010 a revised classification of epileptic conditions (Berg et al, Epilepsia, 51(4):676-685) According to said classification, epileptic conditions may be classified according to the seizure type (generalized seizures, focal seizures, or spasms), etiology (genetic [including idiopathic], structural/metabolic [or symptomatic], or unknown cause [or cryptogenic]), age at onset, cognitive and developmental antecedents and consequences, motor and sensory examinations, EEG features, provoking or triggering factors, and/or patterns of seizure occurrence with respect to sleep. Examples of epileptic conditions include, but are not limited to, epileptic encephalopathies, early infantile epileptic encephalopathies (EIEEs), Dravet syndrome, benign familial neonatal epilepsy (BFNE), early myoclonic encephalopathy (EME),Ohtahara syndrome, epilepsy of infancy with migrating focal seizures, West syndrome, Myoclonic epilepsy in infancy (MEI), benign infantile epilepsy, benign familial infantile epilepsy, myoclonic encephalopathy in non-progressive disorders, febrile seizures plus (FS+), Panayiotopoulos syndrome, epilepsy with myoclonic atonic seizures, benign epilepsy with centrotemporal spikes (BECTS), autosomal-dominant nocturnal frontal lobe epilepsy (ADNFLE), late onset childhood occipital 5 epilepsy, epilepsy with myoclonic absences, Lennox-Gastaut syndrome, epileptic encephalopathy with continuous spike-and-wave during sleep (CSWS), Landau-Kleffner syndrome (LKS), childhood absence epilepsy (CAE), juvenile absence epilepsy (JAE), juvenile myoclonic epilepsy (JME), epilepsy with generalized tonic-clonic seizures alone, progressive myoclonus epilepsies (PME), autosomal dominant epilepsy with auditory features (ADEAF), focal epilepsies, familial and sporadic epileptic condition, lesional and non-lesional epileptic condition, other familial temporal lobe epilepsies (FTLE) (such as, for example, mesial form of FTLE, familial mesial temporal lobe epilepsy (FMTLE) or familial lateral temporal lobe epilepsy (FLTLE), familial focal epilepsy with variable foci (FFEVF, childhood to adult), familial partial epilepsy with variable foci (FPEVF), benign familial partial epilepsies of childhood, reflex epilepsies, mesial temporal lobe epilepsy with hippocampal sclerosis (MTLE with HS), temporal lobe epilepsy, idiopathic generalized epilepsy (IGE), Rasmussen syndrome, gelastic seizures with hypothalamic hamartoma, hemiconvulsion-hemiplegia-epilepsy, neurocutaneous 20 syndromes (tuberous sclerosis complex, Sturge-Weber and the like), epilepsies attributed to malformations of cortical development, tumor, infection or trauma, benign neonatal seizures (BNS), febrile seizures (FS), generalized epilepsy with febrile seizures plus (GEFS+) and epileptic conditions including specific syndromes such as ADNFLE, FTLE, FFEVF, rolandic epilepsies and malignant migrating partial seizures of infancy.

In one embodiment of the present invention, the epileptic condition is focal epilepsy.

In an alternative embodiment of the present invention, the epileptic condition is generalized epilepsy. In one embodiment of the present invention, the epileptic condition is temporal lobe epilepsy.

In an alternative embodiment of the present invention, the epileptic condition is frontal lobe epilepsy. In one embodiment of the present invention, the epileptic condition is mesial temporal lobe epilepsy with hippocampal sclerosis.

In one embodiment of the present invention, the epileptic condition is focal epilepsy attributed to malformations of cortical development.

In one preferred embodiment, the epileptic condition is drug resistant epilepsy.

According to one embodiment, the method of the present invention is used to estimate the physical parameters in horticultural products, food materials or water. The estimation of physical parameters of these products allows the detection of the processing conditions or the quality of food. Indeed, agricultural materials are assessed by a various number of characteristics including moisture content, maturity, freshness, potential insect control, freezing tolerance, frost sensitivity. For example, these characteristics may be determined through the electric conductivity measurement, which measures the resistance to electric flow.

The present invention further relates to a method for generating a mapping of a physical parameter **P** of an area of a medium **M** using at least two electrodes configured to come into contact with the medium **M.**

According to one embodiment, said mapping method comprises a first step of receiving information concerning a first position $[(x_{1a}, y_{1a},); (x_{1b}, y_{1b,})]$ of the electrodes in a first medium region comprised in the area of the medium **M** being mapped.

According to one embodiment, the mapping method comprises a preliminary step of placing the N electrodes at N different locations into the medium, wherein $N \in [2, 200]$. In this embodiment, the electrodes are paired two and sequential measures are performed for each pair of electrodes.

According to one embodiment, the method of mapping comprises a step of obtaining a first value of the physical parameter **P** of the medium **M** in the first medium region using the method for the estimation of the physical parameter **P** according to any one of the embodiment described hereabove.

According to one embodiment, the mapping method comprises a step of associating the first position of the electrodes $[(x_{1a}, y_{1a},); (x_{1b}, y_{1b},)]$ with the first value of the physical parameter **P** and store these mapping data in a computer-readable medium or According to one embodiment, the mapping method transmits the mapping data to a remote server. According to one embodiment, the steps of the mapping method are repeated for at least one second position of the electrodes $[(x_{2a}, y_{2a},); (x_{2b}, y_{2b},)]$ in a second medium region comprised in the area of the medium (M) being mapped. The step of the mapping method may be iteratively repeated for N position of electrodes pair in N medium region in order to obtain a mapping of the distribution of a physical parameter in a medium. The mapping data registered for N electrodes position, may be used to display graphical representation of the distribution of a physical parameter in the medium under investigation, for example, under the form of intensity graph.

The present invention further relates to a computer program comprising instructions which, when the program is executed by a computer, cause the computer to carry out the steps of the method according to any one of the embodiment described above.

The present invention further relates to a computer-readable medium comprising instructions which, when executed by a computer, cause the computer to carry out the steps of the method according to any one of the embodiment described above. According to one embodiment, the computer-readable medium is a non-transitory computer-readable storage medium.

Computer programs implementing the method of the present invention can commonly be distributed to users on a distribution computer-readable storage medium such as, but not limited to, an SD card, an external storage device, a microchip, a flash memory device, a portable hard drive and software websites. From the distribution medium, the computer programs can be copied to a hard disk or a similar intermediate storage medium. The computer programs can be run by loading the computer instructions either from their distribution medium or their intermediate storage medium into the execution memory of the computer, configuring the computer to act in accordance with the method of this invention. All these operations are well- known to those skilled in the art of computer systems.

The invention is defined by the appended claims.

## EXAMPLES

The present invention is further illustrated by the following examples.

Example 1:

The first example consists in an estimation of electrical conductivity post-mortem in a rat brain.

### *Materials and Methods*

Measurement were performed in the brain of adults 3 month-old Sprague-Dawley rats euthanized using a CO2 gradient in accordance with the European Communities Council Directive of 24 November 1986 (86/609/EEC).

The skull surface was immediately removed post-mortem and a human intracranial SEEG electrode (ref D08-15AM) was implanted vertically in rat brains to deliver local, pulsed biphasic stimulation. Stimulation was performed within few minutes post-mortem in order to avoid post-anoxic tissue degeneration.

Stimulation parameters were I=0.2 mA for current intensity, and T=1 ms per phase for the pulse length. Both hemispheres were successively stimulated. As in the case of our calibrated saline solutions, we used an electrophysiology acquisition system (Biopac MP35, Biopac, CA, USA) to record the induced electric potential in brain tissue.

TABLE I

SALINE SOLUTIONS AND RAT BRAIN PARAMETERS ESTIMATION

| Resistance ($\Omega$) | Double layer capacitance ($\mu$F) | Faradaic impedance ($\Omega$) | **Conductivity (S/m)** |
|---|---|---|---|
| *Calibrated Solution ($\sigma$ = 0.1033)* | | | |
| 2079.5 $\pm$ 20.9 | 1.209 $\pm$ 0.040 | 1652.6 $\pm$ 92.1 | **0.1130 $\pm$ 0.0012** |
| *Calibrated Solution ($\sigma$ = 0.2027)* | | | |
| 1161.2 $\pm$ 15.3 | 1.344 $\pm$ 0.033 | 1676.1 $\pm$ 83.6 | **0.2177 $\pm$ 0.0034** |
| *Calibrated Solution ($\sigma$ = 0.3943)* | | | |
| 720.2 $\pm$ 10.1 | 1.449 $\pm$ 0.032 | 1623.4 $\pm$ 66.1 | **0.3922 $\pm$ 0.0073** |
| *Calibrated Solution ($\sigma$ = 0.5786)* | | | |
| 552.9 $\pm$ 8.4 | 1.435 $\pm$ 0.036 | 1486.9 $\pm$ 47.5 | **0.5636 $\pm$ 0.0124** |
| *Right Rat Brain Hemisphere* | | | |
| 1255.1 $\pm$ 61.5 | 0.546 $\pm$ 0.046 | 1631.2 $\pm$ 145.2 | **0.1194 $\pm$ 0.0064** |
| *Left Rat Brain Hemisphere* | | | |
| 1825.7 $\pm$ 39.4 | 0.522 $\pm$ 0.024 | 1598.0 $\pm$ 77.2 | **0.0808 $\pm$ 0.0019** |

### *Results*

Estimated conductivity values presented in Table I was lower than values reported for grey matter in the literature, possibly because conductivity decreases post-mortem. Given that the electrodes were implanted without knowledge of the exact anatomical position of the contacts, it is also possible that electrodes were located in the white matter, possibly accounting for the lower conductivity. It was observed a lower conductivity in the left hemisphere than the right hemisphere, possibly due to the fact that hemispheres were recorded one after the other, possibly involving post-mortem changes in the biophysical properties of tissue between recordings.

Example 2:

The second example consists in an estimation of electrical conductivity *in-clinico* for epileptic patients.

### *Materials and Methods*

Electrophysiological data was recorded from N=2 epileptic patients undergoing SEEG in the context of pre-surgical evaluation. The brain tissues were stimulated with electric pulse of current I = 0.2 mA. This electrical stimulation is significantly lower than those used typically during stimulation sessions in SEEG (i.e. 1-5 mA). A CE-marked, clinical-grade electrophysiology acquisition system was used (Biopac MP35, Biopac, CA, USA).

Electrophysiological recordings show typical features in terms of amplitude, rhythms and epileptic markers and are used by neurologists to determine if an electrode contact is in the grey or white matter (amplitude and rhythms) and also if the region is epileptic or healthy (epileptic markers). The selection of the stimulated regions was done based on the visual inspection of EEG data in addition of neuroimaging data (pre-implantation MRI and post-implantation CT). Seven regions for the first patient and five regions for the second patient were selected.

Stimulation was delivered to each region using the exact same parameters: 3 trains of pulses of 5 seconds per region, delivered at a frequency of 5 Hz at a current of 0.2 mA, using biphasic, charge-balanced pulses of 500 $\mu$s per phase. The pulse length used *in-clinico* was shorter than in the ex vivo experiments to avoid saturation of the recorded signals (example 1 of the present application). The sampling frequency used was 100 kHz to accurately record the short-duration response of brain tissue to each stimulation pulse (50 samples per phase). The total number of pulses was 25 pulses/train (75 pulses for 3 trains). The responses were statistically reproducible from one pulse to another.

The first patient was recorded the day following implantation of SEEG electrodes, and seven regions were recorded. From electrophysiological recordings, electrodes A4-A5 and A9-10 were in the grey matter, while CR4- CR5 were in the white matter. B'3-B'4, TP1-TP2, B1-B2 and TP3-TP4 were in regions generating significant epileptiform activity. The second patient was recorded eight days after implantation, and five regions were recorded. From SEEG signals, it was evaluated that electrodes OF11-OF12 and B1-B2 were in the grey matter, H1-H2 in the white matter, while TB'3-TB'4 and TP'1-TP'2 were in epileptogenic zones.

### Results

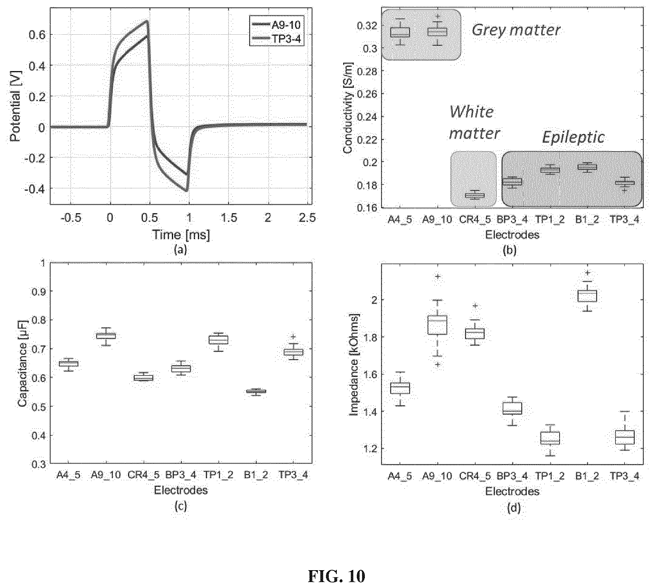

The results for Patient 1 are presented in Figure 10. In Figure 10 (a) two different clusters of conductivity values are observable, one from TP3-TP4 (epileptogenic) and A9-10 (healthy). The amplitude difference in the recorded response to pulse stimulation is related to their different conductivity (approx. factor 2). Figure 10 (b) presents conductivity values for the seven electrodes. Regions A4-A5 and A9-A10, located in the grey matter, were estimated to have a conductivity of 0.31 S/m and CR4-CR5 a conductivity of 0.17 S/m, respectively. These values did not depend on the epileptogenicity of recorded brain regions.

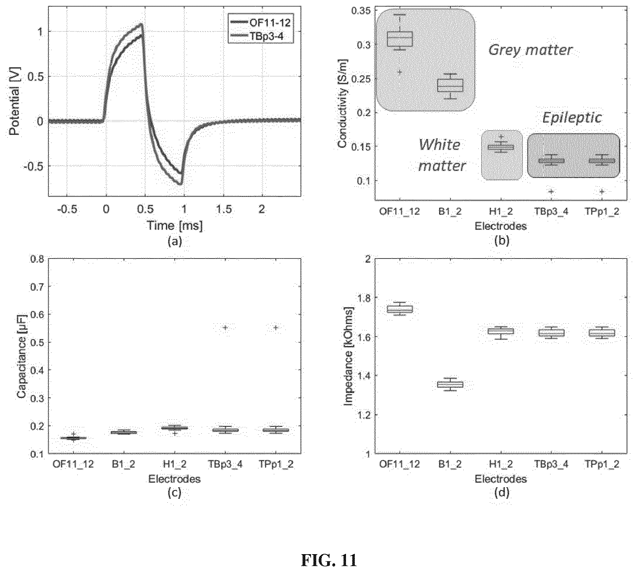

Results for Patient 2 are presented in Figure 11. Fig. 11 (a) presents two different responses to pulsed stimulation, one from an epileptic zone (TB'3-TB'4) and from a healthy zone OF11-OF12. The waveforms are slightly different than for Patient 1. Figure 11 (b) presents estimated conductivity values for the five pairs of electrode contacts. Similarly to Patient 1, two clusters of conductivity values emerge, depending on whether electrode contacts were in the grey or white matter, with regions identified as epileptic having the lowest conductivity values. Estimated values for the double layer capacitance and faradaic impedance were 0.2 $\mu$F and 1.3-1.8 k$\Omega$. While the faradaic impedance estimation is in excellent agreement with the value obtained for Patient 1, the double layer capacitance is notably lower for Patient 2, possibly due to CSF infiltration post-surgery given the delay between electrodes implantation and recording. Since the electrode-electrolyte interface depends on factors such as local gliosis or infiltration of cerebrospinal fluid, there is no reason *a priori* that epileptogenic regions have differences in the $Z_f$ and $C_{dl}$ parameters; and will depend mostly of the day after implantation.

Example 3:

The third example consists in an estimation of electrical conductivity of a saline solution.

### Materials and Methods

A clinical electrode (DIXI Microtechniques, Besançon, France) was placed into a solution with different calibrated electrical conductivities (4 solutions with electric conductivity values of 0.1033, 0.2027, 0.3943, and 0.5786 S/m, respectively). It was used a clinical-grade stimulator (S12X, Grass Technologies, Natus Medical Inc., USA) to deliver biphasic, charge-balanced pulse electrical stimulation with an intensity I=0.2 mA stimulating current (the lowest available on this stimulator, to avoid input saturation) and a pulse length of T=1 ms per phase (total pulse length of 2 ms). An electrophysiology acquisition system (Biopac MP35, Biopac, CA, USA) was used to record the electric potential induced in the solution during stimulation.

### Results

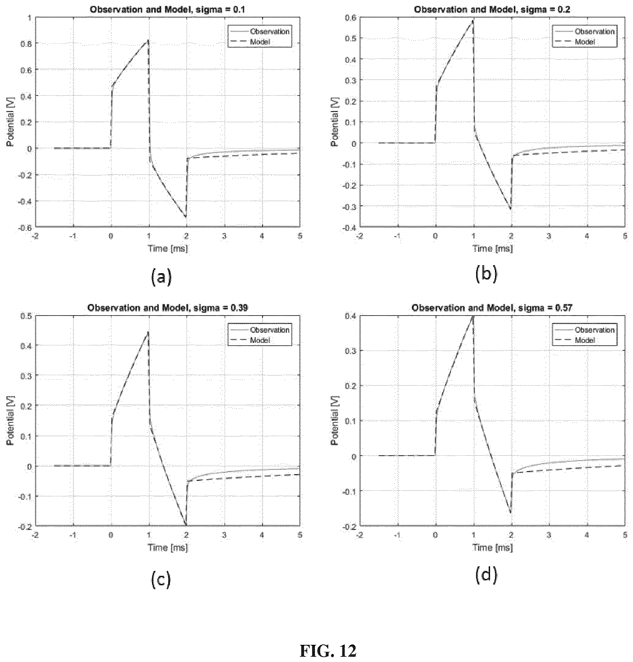

The recorded time course of the electric potential depends strongly on electrical conductivity. Waveform discontinuities convey information on electrical conductivity, and conductivity increases as the medium resistance decreases. Importantly, the predefined analytic model parameters depend on a number of physical phenomena, among which the ion redistribution. It was assumed that, for small changes in conductivity, model parameters remain unchanged. We compare in Figure 12 the agreement between the model **M(t)** and experimental recordings in saline solutions.

The predefined analytic model reproduces accurately the time course of the recorded potential for both positive and negative phases. On average, the predefined analytic model estimation of electrical conductivity was within 2% of the ground truth value.

### REFERENCES

1 - System;
2 - Electrodes;
3 - Current generator;
4 - Computer-readable memory;
5 - Acquisition unit;
6 - Processor;

61 - Stimulation module;

62 - Acquisition module;

63 - Calculation module;

7 - User interface;

FIT - Step of fitting the measurement of the electric potential variation as a function of time;

GE - Geometry specifications of the electrodes;

I - Current;

M - Medium;

M(t) - Predefined analytic model;

OUT - Step of outputting a value of the physical parameter obtained from the fitting;

P - Physical parameter;

REC - Step of receiving a measurement of an electric potential variation as a function of time;

Rm - electrical resistance;

T - Pulse duration;

Tm - Time window;

Ts - Stimulation duration;

$\Delta V(t)$ - Electric potential variation as a function of time;

$\sigma$ - Electrical conductivity.

**Claims**

1. A system (1) for the estimation of at least one physical parameter (P) of a region of a biological and/or physiological medium (M) comprising at least one electrolyte, said system comprising:

   - at least two electrodes (2), of which at least one working electrode and at least one counter electrode, configured to come into contact with the region of the medium (M);
   - a current generator (3) configured to deliver to the electrodes (2) a train of electric pulses of current (I), each electric pulse having a pulse duration (T);
   - a computer-readable memory (4) comprising at least one predefined analytic model (M(t)) of an electric potential, between the working electrode and the counter electrode, as a function of time, receiving as inputs at least the current (I) and the pulse duration (T) and comprising at least one physical parameter (P) of the medium (M) to be estimated;
   - an acquisition unit (5) comprising a signal amplifier configured to acquire and amplify an electric potential recorded by the electrodes; and
   - a processor (6) comprising:

     • a stimulation module (61) configured to control the current generator (3) so as to deliver a biphasic charge-balanced current, comprising electric pulses, during a stimulation duration (Ts);
     • an acquisition module (62) configured to trigger an acquisition of an electric potential variation as a function of time ($\Delta V(t)$) during a time window (Tm) comprised in the stimulation duration (Ts); and
     • a calculation module (63) configured to receive the acquired electric potential variation of the region of the medium (M) between the working electrode and the counter electrode as a function of time ($\Delta V(t)$), fit the acquired electric potential variation as a function of time ($\Delta V(t)$) using the predefined analytic model (M(t)) retrieved from the computer-readable memory, and output a value of the physical parameter (P) obtained from the fitting of the predefined analytic model (M(t));

   said system **characterized in that**
   the predefined analytic model (M(t)) is obtained from the coupling of an analytical model of the electric field generated by the electrodes (2) with a double layer model generated at the electrode-medium interface, said coupling accounting for contributions from the electrode-electrolyte interface.

2. The system according to claim 1, wherein the at least two electrodes (2) are bipolar cylindrical or plate electrodes.

3. The system according to either claim 1 or claim 2, wherein the electrodes (2) are configured for insertion in a region of the medium (M).

4. The system according to any one of claims 1 to 3, wherein the stimulation module (61) controls the current generator (3) so as to deliver electrical pulses having a current (I) that does not saturate the signal amplifier.

5. A method for the local estimation of at least one physical parameter of a region of a biological and/or physiological medium (M) comprising at least one electrolyte, said method comprising the steps of:

- receiving (REC) a measurement of an electric potential variation as a function of time (ΔV(t)) in a time window (Tm), during which biphasic charge-balanced electrical pulses are delivered to the region of the medium (M) by at least two electrodes (2), of which at least one working electrode and at least one counter electrode, configured to come into contact with the region of the medium (M), wherein each electric pulse has a pulse duration (T);
- fitting (FIT) the measurement of the electric potential variation of the region of the medium (M) between the working electrode and the counter electrode as a function of time (ΔV(t)) using a predefined analytic model (M(t)) of the electric potential as a function of time, wherein the predefined analytic model (M(t)) receives as inputs at least the current (I) and the pulse duration (T) and comprises at least one physical parameter (P) of the region of the medium (M);
- outputting (OUT) a value of the physical parameter (P) obtained from the fitting of the predefined analytic model (M(t));
said method **characterized in that**
the predefined analytic model (M(t)) is obtained from the coupling of an analytical model of the electric field generated by the electrodes (2) with a double layer model generated at the electrode-medium interface, said coupling accounting for contributions from the electrode-electrolyte interface.

6. The method according to claim 5, wherein the physical parameter (P) is associated to the electrical resistance (Rm) of the region of the medium (M) in which the electrodes are intended to be located.

7. The method according to claim 6, further comprising a step of receiving geometry specifications of the electrodes (GE) and using said geometry specifications of the electrodes (GE) and the electrical resistance (Rm) of the region of the medium (M) to calculate (CAL) the electrical conductivity (σ) of the region of the medium (M).

8. The method according to any one of claims 5 to 7, wherein the medium (M) are brain tissues.

9. The method according to any one of claims 5 to 8, wherein the value of the physical parameter (P) is compared to a predefined threshold.

10. The method according to any one of claims 5 to 9, wherein the electric potential variation received is measured with a sampling frequency superior to 8 kHz.

11. A method for generating a mapping of a physical parameter (P) of an area of a medium (M), comprising at least one electrolyte, using at least two electrodes configured to come into contact with the medium (M), said method comprising steps of:

- receiving information concerning a first position of the electrodes in a first region of the medium (M) comprised in the area of the medium (M) being mapped;
- obtaining a first value of the physical parameter (P) of the medium (M) in the first region of the medium (M) according to the method of any one of claims 5 to 10; and
- associating and registering the first position of the electrodes with the first value of the physical parameter (P);

wherein the steps of the method are repeated for at least one second position of the electrodes in a second region of the medium (M) comprised in the area of the medium (M) being mapped.

12. A computer program comprising instructions which, when the program is executed by a computer, cause the computer to carry out the steps of the method according to any one of claims **5** to **11.**

13. A computer-readable medium comprising instructions which, when executed by a computer, cause the computer to carry out the steps of the method according to any one of claims **5** to **11.**

**Patentansprüche**

1. System (1) zur Schätzung mindestens eines physikalischen Parameters (P) einer Region eines biologischen und/oder physiologischen Mediums (M), das mindestens einen Elektrolyten umfasst, wobei das System umfasst:

   - mindestens zwei Elektroden (2), davon mindestens eine Arbeitselektrode und mindestens eine Gegenelektrode, die so konfiguriert sind, dass sie mit der Region des Mediums (M) in Kontakt kommen;
   - einen Stromgenerator (3), der so konfiguriert ist, dass er an die Elektroden (2) eine Folge von elektrischen Stromimpulsen (I) abgibt, wobei jeder elektrische Impuls eine Impulsdauer (T) aufweist;
   - einen computerlesbaren Speicher (4), der mindestens ein vordefiniertes analytisches Modell (M(t)) eines elektrischen Potentials zwischen der Arbeitselektrode und der Gegenelektrode in Abhängigkeit von der Zeit umfasst, das als Eingänge mindestens den Strom (I) und die Impulsdauer (T) empfängt und mindestens einen physikalischen Parameter (P) des Mediums (M), der geschätzt werden soll, umfasst;
   - eine Erfassungseinheit (5), die einen Signalverstärker umfasst, der so konfiguriert ist, dass er ein elektrisches Potential, das von den Elektroden aufgezeichnet wird, erfasst und verstärkt; und
   - einen Prozessor (6), umfassend:

     -- ein Stimulationsmodul (61), das so konfiguriert ist, dass es den Stromgenerator (3) so steuert, dass er während einer Stimulationsdauer (Ts) einen zweiphasigen, ladungsausgeglichenen Strom abgibt, der elektrische Impulse umfasst;
     -- ein Erfassungsmodul (62), das so konfiguriert ist, dass es eine Erfassung einer elektrischen Potentialschwankung in Abhängigkeit von der Zeit (ΔV(t)) während eines Zeitfensters (Tm), das in der Stimulationsdauer (Ts) umfasst ist, auslöst; und
     -- ein Berechnungsmodul (63), das so konfiguriert ist, dass es die erfasste elektrische Potentialschwankung der Region des Mediums (M) zwischen der Arbeitselektrode und der Gegenelektrode in Abhängigkeit von der Zeit (ΔV(t)) empfängt, die erfasste elektrische Potentialschwankung in Abhängigkeit von der Zeit (ΔV(t)) unter Verwendung des vordefinierten analytischen Modells (M(t)), das aus dem computerlesbaren Speicher abgerufen wird, anpasst und einen Wert des physikalischen Parameters (P), der aus der Anpassung des vordefinierten analytischen Modells (M(t)) erhalten wird, ausgibt;
     wobei das System **dadurch gekennzeichnet ist, dass**
     das vordefinierte analytische Modell (M(t)) aus der Kopplung eines analytischen Modells des elektrischen Feldes, das von den Elektroden (2) erzeugt wird, mit einem Doppelschichtmodell erhalten wird, das an der Elektroden-Medium-Grenzfläche erzeugt wird, wobei die Kopplung Beiträge von der Elektroden-Elektrolyt-Grenzfläche berücksichtigt.

2. System nach Anspruch **1,** wobei die mindestens zwei Elektroden (2) bipolare zylindrische oder Plattenelektroden sind.

3. System nach entweder Anspruch **1** oder Anspruch **2,** wobei die Elektroden (2) zum Einführen in eine Region des Mediums (M) konfiguriert sind.

4. System nach einem der Ansprüche **1** bis **3,** wobei das Stimulationsmodul (61) den Stromgenerator (3) so steuert, dass er elektrische Impulse abgibt, die einen Strom (I) aufweisen, der den Signalverstärker nicht sättigt.

5. Verfahren zur lokalen Schätzung mindestens eines physikalischen Parameters einer Region eines biologischen und/oder physiologischen Mediums (M), das mindestens einen Elektrolyten umfasst, wobei das Verfahren die folgenden Schritte umfasst:

   - Empfangen (REC) einer Messung einer elektrischen Potentialschwankung in Abhängigkeit von der Zeit (ΔV(t)) in einem Zeitfenster (Tm), während dem zweiphasige ladungsausgeglichene elektrische Impulse an die Region des Mediums (M) durch mindestens zwei Elektroden (2) abgegeben werden, davon mindestens eine Arbeitselektrode und mindestens eine Gegenelektrode, die so konfiguriert sind, dass sie mit der Region des Mediums (M) in Kontakt kommen, wobei jeder elektrische Impuls eine Impulsdauer (T) aufweist;
   - Anpassen (FIT) der Messung der elektrischen Potentialschwankung der Region des Mediums (M) zwischen der Arbeitselektrode und der Gegenelektrode in Abhängigkeit von der Zeit (ΔV(t)) unter Verwendung eines vordefinierten analytischen Modells (M(t)) des elektrischen Potentials in Abhängigkeit von der Zeit, wobei das vordefinierte analytische Modell (M(t)) als Eingänge mindestens den Strom (I) und die Impulsdauer (T) empfängt und mindestens einen physikalischen Parameter (P) der Region des Mediums (M) umfasst; und

- Ausgeben (OUT) eines Wertes des physikalischen Parameters (P), der aus der Anpassung des vordefinierten analytischen Modells (M(t)) erhalten wird;
wobei das Verfahren **dadurch gekennzeichnet ist, dass**
das vordefinierte analytische Modell (M(t)) aus der Kopplung eines analytischen Modells des elektrischen Feldes, das von den Elektroden (2) erzeugt wird, mit einem Doppelschichtmodell erhalten wird, das an der Elektroden-Medium-Grenzfläche erzeugt wird, wobei die Kopplung Beiträge von der Elektroden-Elektrolyt-Grenzfläche berücksichtigt.

6. Verfahren nach Anspruch **5,** wobei der physikalische Parameter (P) mit dem elektrischen Widerstand (Rm) der Region des Mediums (M), in dem die Elektroden angebracht werden sollen, verknüpft ist.

7. Verfahren nach Anspruch **6,** das weiter einen Schritt des Empfangens von Geometriespezifikationen der Elektroden (GE) und Verwendens der Geometriespezifikationen der Elektroden (GE) und des elektrischen Widerstands (Rm) der Region des Mediums (M) umfasst, um die elektrische Leitfähigkeit ($\sigma$) der Region des Mediums (M) zu berechnen (CAL).

8. Verfahren nach einem der Ansprüche **5** bis **7,** wobei das Medium (M) Hirngewebe sind.

9. Verfahren nach einem der Ansprüche **5** bis **8,** wobei der Wert des physikalischen Parameters (P) mit einer vordefinierten Schwelle verglichen wird.

10. Verfahren nach einem der Ansprüche **5** bis **9,** wobei die elektrische Potentialschwankung, die empfangen wird, mit einer Abtastfrequenz von über 8 kHz gemessen wird.

11. Verfahren zum Erzeugen einer Abbildung eines physikalischen Parameters (P) eines Bereichs eines Mediums (M), das mindestens einen Elektrolyten umfasst, unter Verwendung von mindestens zwei Elektroden, die so konfiguriert sind, dass sie mit dem Medium (M) in Kontakt kommen, wobei das Verfahren die folgenden Schritte umfasst:

- Empfangen von Informationen, die eine erste Position der Elektroden in einer ersten Region des Mediums (M) betreffen, die in dem Bereich des Mediums (M), der abgebildet wird, umfasst ist;
- Erhalten eines ersten Wertes des physikalischen Parameters (P) des Mediums (M) in der ersten Region des Mediums (M) nach dem Verfahren nach einem der Ansprüche **5** bis **10;** und
- Verknüpfen und Registrieren der ersten Position der Elektroden mit dem ersten Wert des physikalischen Parameters (P);

wobei die Schritte des Verfahrens für mindestens eine zweite Position der Elektroden in einer zweiten Region des Mediums (M), die in dem Bereich des Mediums (M), der abgebildet wird, umfasst ist, wiederholt werden.

12. Computerprogramm, das Anweisungen umfasst, die, wenn das Programm von einem Computer ausgeführt wird, bewirken, dass der Computer die Schritte des Verfahrens nach einem der Ansprüche **5** bis **11** ausführt.

13. Computerlesbares Medium, das Anweisungen umfasst, die, wenn sie von einem Computer ausgeführt werden, bewirken, dass der Computer die Schritte des Verfahrens nach einem der Ansprüche **5** bis **11** ausführt.

## Revendications

1. Système (1) pour l'estimation d'au moins un paramètre (P) d'une région d'un milieu biologique et/ou physiologique (M), comprenant au moins un électrolyte, ledit système comprenant :

- au moins deux électrodes (2), parmi lesquelles au moins une électrode de travail et au moins une contre-électrode, configurées pour venir en contact avec la région du milieu (M) ;
- un générateur de courant (3), configuré pour délivrer aux électrodes (2) un train d'impulsions électriques (I), chaque impulsion électrique ayant une durée d'impulsion (T) ;
- une mémoire lisible par ordinateur (4), comprenant au moins un modèle analytique prédéfini (M(t)) d'un potentiel électrique entre l'électrode de travail et la contre-électrode, fonction du temps, recevant en entrée au moins le courant (I) et la durée d'impulsion (T) et comprenant au moins un paramètre physique (P) du milieu (M) à estimer ;
- une unité d'acquisition (5), comprenant un amplificateur de signal configuré pour acquérir et amplifier un potentiel électrique enregistré par les électrodes ; et
- un processeur (6), comprenant :
- un module de stimulation (61), configuré pour contrôler le générateur de courant (3) pour délivrer un courant biphasique à charge équilibrée comprenant des impulsions électriques pendant une durée de stimulation (Ts) ;
- un module d'acquisition (62) configuré pour déclencher une acquisition d'une variation de potentiel électrique en fonction du temps (ΔV(t)) pendant une fenêtre temporelle (Tm) comprise dans la durée de stimulation (Ts) et
- un module de calcul (63) configuré pour recevoir la variation de potentiel électrique acquise de la région du milieu (M) entre l'électrode de travail et la contre-électrode en fonction du temps (ΔV(t)), ajuster la variation de potentiel électrique acquise en fonction du temps (ΔV(t)) en utilisant le modèle analytique prédéfini (M(t)) extrait de la mémoire lisible par ordinateur et fournir une valeur du paramètre physique (P) obtenue à partir de l'ajustement du modèle analytique prédéfini (M(t)) ;
ledit système étant **caractérisé en ce que**
le modèle analytique prédéfini (M(t)) est obtenu à partir du couplage d'un modèle analytique du champ électrique généré par les électrodes (2) avec un modèle de double couche généré à l'interface électrode-milieu, ledit couplage prenant en compte des contributions de l'interface électrode-électrolyte.

2. Système selon la revendication **1,** dans lequel les au moins deux électrodes (2) sont des électrodes bipolaires cylindriques ou plates.

3. Système selon la revendication **1** ou la revendication **2,** dans lequel les électrodes (2) sont configurées pour insertion dans une région du milieu (M).

4. Système selon l'une des revendications **1** à **3,** dans lequel le module de stimulation (61) contrôle le générateur de courant (3) de sorte à délivrer des impulsions électriques ayant un courant (I) qui ne sature pas l'amplificateur de signal.

5. Procédé pour l'estimation locale d'au moins un paramètre physique d'une région d'un milieu biologique et/ou physiologique (M) comprenant au moins un électrolyte, ledit procédé comprenant les étapes :

- recevoir (REC) une mesure d'une variation de potentiel électrique en fonction du temps (ΔV(t)) dans une fenêtre temporelle (Tm) durant laquelle des impulsions électriques biphasiques à charge équilibrée sont délivrées à la région du milieu (M) par au moins deux électrodes (2), parmi lesquelles au moins une électrode de travail et au moins une contre-électrode, configurées pour venir en contact avec la région du milieu (M), dans lequel chaque impulsion électrique présente une durée d'impulsion (T) ;
- ajuster (FIT) la mesure de la variation de potentiel électrique de la région du milieu (M) entre l'électrode de travail et la contre-électrode en fonction du temps (ΔV(t)) en utilisant un modèle analytique prédéfini (M(t)) du potentiel électrique en fonction du temps, dans lequel le modèle analytique prédéfini (M(t)) reçoit en entrée au moins le courant (I) et la durée d'impulsion (T) et comprend au moins un paramètre physique (P) de la région du milieu (M) et
- fournir (OUT) une valeur du paramètre physique (P) obtenue à partir de l'ajustement du modèle analytique prédéfini (M(t)) ;
ledit procédé étant **caractérisé en ce que**
le modèle analytique prédéfini (M(t)) est obtenu à partir du couplage d'un modèle analytique du champ électrique généré par les électrodes (2) avec un modèle de double couche généré à l'interface électrode-milieu, ledit couplage prenant en compte de contributions de l'interface électrode-électrolyte.

6. Procédé selon la revendication **5,** dans lequel le paramètre physique (P) est associé à la résistance électrique (Rm) de la région du milieu (M) dans lequel les électrodes sont conçues pour être localisées.

7. Procédé selon la revendication **6,** comprenant en outre une étape de réception de spécifications géométriques des électrodes (GE) et d'utilisation desdites spécifications géométriques (GE) et de la résistance électrique (Rm) de la région du milieu (M) pour calculer (CAL) la conductivité électrique (σ) de la région du milieu (M).

8. Procédé selon l'une des revendications **5** à **7,** dans lequel le milieu (M) comprend des tissus cérébraux.

9. Procédé selon l'une des revendications **5** à **8,** dans lequel la valeur du paramètre physique (P) est comparée à un seuil prédéfini.

10. Procédé selon l'une des revendications **5** à **9,** dans lequel la variation de potentiel électrique reçue est mesurée avec une fréquence d'échantillonnage supérieure à 8 kHz.

11. Procédé pour générer une cartographie d'un paramètre physique (P) d'une zone d'un milieu (M) comprenant au moins un électrolyte, en utilisant au moins deux électrodes configurées pour venir en contact avec le milieu (M), ledit procédé comprenant les étapes :

- recevoir une information concernant une première position des électrodes dans une première région du milieu (M) comprise dans la zone du milieu (M) étant cartographiée ;
- obtenir une première valeur du paramètre physique (P) du milieu (M) dans la première région du milieu (M) selon le procédé de l'une quelconque des revendications **5** à **10** ; et
- associer et enregistrer la première position des électrodes avec la première valeur du paramètre physique (P) ;

dans lequel les étapes du procédé sont répétées pour au moins une deuxième position des électrodes dans une deuxième région du milieu (M) comprise dans la zone du milieu (M) étant cartographiée.

12. Programme d'ordinateur, comprenant des instructions qui, lorsque le programme est exécuté par un ordinateur, entraîne l'ordinateur à exécuter les étapes du procédé selon l'une quelconque des revendications **5** à **11.**

13. Support lisible par ordinateur, comprenant des instructions qui, lorsqu'elles sont exécutées par un ordinateur, entraîne l'ordinateur à exécuter les étapes du procédé selon l'une quelconque des revendications **5** à **11.**

FIG. 1

FIG. 2

FIG. 3

FIG. 4

**FIG. 5**

**FIG. 6**

**FIG. 7**

**FIG. 8**

**FIG. 9**

**FIG. 10**

FIG. 11

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014303691 A1

**Non-patent literature cited in the description**

- **BERG et al.** *Epilepsia*, vol. 51 (4), 676-685